(19) **Europäisches Patentamt**
**European Patent Office**
**Office européen des brevets**

(11) **EP 2 175 030 B1**

(12) **EUROPEAN PATENT SPECIFICATION**

(45) Date of publication and mention
of the grant of the patent:
**26.12.2018 Bulletin 2018/52**

(51) Int Cl.:
**C12P 7/64** (2006.01) **C11C 1/08** (2006.01)
**C11C 3/02** (2006.01)

(21) Application number: **08826987.3**

(22) Date of filing: **08.08.2008**

(86) International application number:
**PCT/JP2008/002179**

(87) International publication number:
**WO 2009/019893 (12.02.2009 Gazette 2009/07)**

(54) **METHOD FOR PRODUCING DIACYLGLYCEROL-RICH FAT AND/OR OIL**

VERFAHREN ZUR HERSTELLUNG VON DIACYLGLYCEROL-REICHEM FETT UND/ODER ÖL

PROCÉDÉ DE FABRICATION D'HUILE ET/OU GRAISSE RICHE EN DIACYLGLYCÉROL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **08.08.2007 JP 2007206413**

(43) Date of publication of application:
**14.04.2010 Bulletin 2010/15**

(73) Proprietor: **Kao Corporation**
**Chuo-Ku**
**Tokyo 103-8210 (JP)**

(72) Inventors:
• **KASE, Minoru**
**Kamisu-shi**
**Ibaraki 314-0103 (JP)**
• **KOMATSU, Toshiteru**
**Kamisu-shi**
**Ibaraki 314-0103 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 717 318      EP-A1- 2 287 325**
**JP-A- 2007 014 263**

• **KRISTENSEN J.B. ET AL.: 'Process Optimization Using Response Surface Design and Pilot Plant Production of Dietary Diacylglycerols by Lipase-Catalyzed Glycerolysis' J. AGRIC. FOOD CHEM. vol. 53, 2005, pages 7059 - 7066, XP002402019**
• **CERIANI R. ET AL.: 'Simulation of Batch Physical Refining and Deodorization Processes' JAOCS vol. 81, no. 3, 2004, pages 305 - 312, XP001525548**
• **SUGIMOTO I. ET AL.: 'Saishin no Shishitsu Eiyo to Yushi Shokuhin Kagaku Seiyu Oyobi Yushi Shokuhin no Kako Gijutsu ni Shinpo Yushi no Dasshu to Shokuyoabura no Anteisei' NIPPON YUKA GAKKAISHI vol. 48, no. 10, 1999, pages 1123 - 1131, XP008147059**
• **MARUYAMA T. ET AL.: 'Shokubutuabura no Seisei Kotei ni Okeru Trans-fatty Acid no Seisei Oyobi Shokuyo Shokubutsu Yushi ni Fukumareru Trans-fatty Acid ni Tsuite' NIPPON YUKA GAKKAISHI vol. 48, no. 9, 1999, pages 877 - 883**
• **OTA S. ET AL.: 'Shokuyo Yushi no Gijutsuteki Henkan VII Seisei Kotei 8' SHOKUHIN KIKAI SOCHI 1989, pages 73 - 80, XP008129224**
• **OTA S. ET AL.: 'Shokuyo Yushi no Gijutsuteki Henkan VII Seisei Kotei 7' SHOKUHIN KIKAI SOCHI 1989, pages 70 - 79, XP008129225**
• **OTA S. ET AL.: 'Shokuyo Yushi no Gijutsuteki Henkan VII Seisei Kotei 6' SHOKUHIN KIKAI SOCHI 1989, pages 76 - 83, XP008129226**
• **SCHWARZ W. ET AL.: 'Formation of trans polyalkenoic fatty acids during vegetable oil refining' EUR. J. LIPID. SCI. TECHNOL. vol. 102, no. 10, 2000, pages 648 - 649, XP008129227**

**(Cont. next page)**

- LONG K. ET AL.: 'Physico-chemical properties of palm olein fractions as a function of diglyceride content in the starting material' EUR. J. LIPID. SCI. TECHNOL. vol. 107, no. 10, 2005, pages 754 - 761, XP008129228

**Description**

FIELD OF THE INVENTION

The present invention relates to a method for producing a diacylglycerol-rich fat and/or oil.

BACKGROUND OF THE INVENTION

A diacylglycerol-rich fat and/or oil has physiological actions such as a body fat burning action, and thus are widely used as edible oils. The diacylglycerol-rich fat and/or oil that is produced by a conventional method contains impurities such as fatty acids, monoacylglycerols and odorous components, and in order to use a diacylglycerol-rich fat and/or oil as an edible oil, it is necessary to improve the flavor by eliminating these impurities. Therefore, the so-called deodorization operation, which involves contacting a fat and/or oil with steam under high temperature and low pressure, is generally carried out (Patent Document 1).

In conventional deodorization operations, if the temperature is low, a diacylglycerol-rich fat and/or oil has unpleasant flavor and have residual fatty acids and monoacylglycerols, because the effect of distilling off the impurities is insufficient. On the other hand, if the temperature is increased to distill off the impurities, a disproportionation reaction or the like occurs, and consequently, monoacylglycerols and triacylglycerols are generated, resulting in a decrease in the diacylglycerol content. This could also pose a problem that trans-unsaturated fatty acids increase in the amount.

As a way to produce a diacylglycerol-rich fat and/or oil that has a small content of trans-unsaturated fatty acids, there is a method of hydrolyzing a raw fat and/or oil through a combination of a high pressure splitting method and an enzymatic splitting method to produce fatty acids, and esterifying the fatty acids and glycerin (Patent Document 2). There is also a method of producing an edible fat and/or oil having a low content of trans-fatty acids, by deodorizing the fat and/or oil using a thin layer type apparatus and a tray type apparatus (Patent Document 3).

> Patent Document 1: JP-B-3-7240
> Patent Document 2: JP-A-2006-137923
> Patent Document 3: JP-A-2007-14263

DISCLOSURE OF THE INVENTION

The present invention provides a method for producing a diacylglycerol-rich fat and/or oil, comprising hydrolyzing a raw fat and/or oil by an enzymatic splitting method to obtain a fatty acid, and then esterifying the fatty acid and a glycerin, wherein the raw fat and/or oil containing an undeodorized fat and/or oil in an amount of 50% by weight or more is used, and a deodorization treatment, which is an operation of removing any odorous components by performing a reduced pressure steam distillation under heating, is carried out after the esterification reaction, to the extent that the relationship between the deodorization time (x) and the deodorization temperature (y) fall in the range defined by the following (i) and (ii):

$$\text{(i)} \quad y >= -2.59x + 566$$

$$\text{(ii)} \quad y <= 617x^{-0.0455},$$

provided that $475 <= y <= 563$,
wherein x represents the deodorization time (minutes), and y represents the deodorization temperature (K), and wherein the deodorization treatment is carried out at a pressure ranging from 0.01 to 4 kPa.

DETAILED DESCRIPTION OF THE INVENTION

An investigation was made on a method of suppressing the generation of trans-unsaturated fatty acids during the production of a diacylglycerol-rich fat and/or oil, and in the method of the Patent Document 2, it was found that such suppression is not necessarily sufficient because the high pressure splitting method is concurrently carried out upon conducting the hydrolysis of the raw fat and/or oil. It was also found that if the enzymatic splitting method is solely carried out upon conducting the hydrolysis of the raw fat and/or oil, the diacylglycerol obtained by subjecting the resulting fatty acids and glycerin to an enzymatic esterification reaction is far from satisfactory in terms of flavor or color.

In the deodorization operation of a diacylglycerol-rich fat and/or oil, it has been necessary to carry out the deodorization operation at a medium temperature, in part because the purity of diacylglycerols needs to stay high, in part because the rate of generation of trans-unsaturated fatty acids is increased due to an elevated temperature. However, it was found that the obtained fat and/or oil, depending on the conditions for deodorization, is not necessarily satisfactory in terms of the flavor characteristic of diacylglycerols (e.g., the umami taste and rich body taste) and, what is more, it proved impossible to suppress the generation of trans-unsaturated fatty acids.

On the other hand, unlike the deodorization of a triglyceride-rich fat and/or oil, the deodorization of a diacylglycerol-rich fat and/or oil has such a problem that the production of monoacylglycerol (impurity) generated by the disproportionation reaction during a deodorization process could compete against the removal of monoacylglycerols by distillation. Furthermore, diacylglycerols are less hydrophobic as compared with triacylglycerols and therefore exhibit high affinity to fatty acids and monoacylglycerols, so that it is difficult to remove them by distillation.

As such, as compared with triacylglycerols, a diacylglycerol-rich fat and/or oil has drawbacks that the fat and/or oil yield is largely decreased, and that the trans-unsaturated fatty acid content increases, concomitantly with an increase in the rate of removal of impurities.

Thus, the present invention provides a method for efficiently producing a diacylglycerol- rich fat and/or oil, which has a low content of trans-unsaturated fatty acids and has good color and good flavor.

The inventors of the present invention have conducted extensive investigations on the deodorization operation of a diacylglycerol-rich fat and/or oil, and they found that when an esterification reaction is carried out using a raw fat and/or oil having an undeodorized fat and/or oil content of 50% by weight or more, and then a deodorization treatment is carried out such that the deodorization time and the deodorization temperature fall within a certain range, a diacylglycerol-rich fat and/or oil that has a low content of trans-unsaturated fatty acids and improved color and flavor, is obtained with high efficiency.

According to the present invention, a diacylglycerol-rich fat and/or oil that has a low content of trans-unsaturated fatty acids and has good color and good flavor, can be produced with high efficiency.

In the present invention, a raw fat and/or oil containing undeodorized fat and/or oil in an amount of 50% by weight or more (hereinafter, simply indicated as "%") is used from the viewpoints of lowering the content of trans-unsaturated fatty acids among the constituent fatty acids of the fat and/or oil, and improving the flavor such as umami taste and rich body taste. The content of the undeodorized fat and/or oil is more preferably 60% or more, and even more preferably 75 to 100%. The term "undeodorized fat and/or oil" as used in the present invention means an fat and/or oil that has not been subjected to deodorization in the course of a purification treatment of a raw fat and/or oil material. The term "deodorization" means an operation of removing any odorous components by performing a reduced pressure steam distillation under heating.

The raw fat and/or oil may be either a plant fat and/or oil, or an animal fat and/or oil. Specific examples of the raw material include rapeseed oil, sunflower oil, corn oil, soybean oil, rice bran oil, safflower oil, cotton seed oil, beef tallow, linseed oil, fish oil, and the like. Products prepared by fractionating or mixing these fats and/or oils, and products having the fatty acid composition adjusted by hydrogenation, trans-esterification reaction or the like, can also be utilized as the raw material, but products that have not been subjected to hydrogenation are preferable, from the viewpoint of lowering the content of trans-unsaturated fatty acids among the constituent fatty acids of the fat and/or oil.

It is preferable to eliminate from the raw fat and/or oil any solids other than the oil components by filtration, centrifugation or the like, after the process of pressing oil from the respective raw material plant or animal. Next, it is preferable to add and mix water, and additionally an acid in some cases, into the raw fat and/or oil, and then to separate the gum fraction by centrifugation or the like to thereby degum the fat and/or oil. It is also preferable to subject the raw fat and/or oil to deacidification by adding and mixing an alkali into the raw fat and/or oil, and then performing water washing and dehydration. Furthermore, from the viewpoint of reducing the sensation of stimulation of a diacylglycerol-rich fat and/or oil, it is preferable to subject the raw fat and/or oil to decoloration by contacting the raw fat and/or oil with an adsorbent such as activated white clay, and then separating the adsorbent by filtration or the like. These treatments are preferably carried out in an order as described above, but the order may be modified. In addition to these, the raw fat and/or oil may also be subjected to wintering, which separates solids at low temperature, for the removal of waxes. It is also acceptable to use a fat and/or oil that has been deodorized, as a part of the raw fat and/or oil.

According to the present invention, it is preferable to use a raw fat and/or oil in which the content of trans-unsaturated fatty acids among the constituent fatty acids is 1.5% or less, more preferably 1% or less, and far more preferably 0.5% or less, from the viewpoint of lowering the content of trans-unsaturated fatty acids among the constituent fatty acids in the fatty acids obtainable after hydrolysis. Incidentally, the content of trans-unsaturated fatty acids among the constituent fatty acids in the case of using two or more types of fats and/or oils, is the content in the total amount of the fat and/or oil (indicated in Examples).

According to the present invention, a raw fat and/or oil containing undeodorized fat and/or oil in an amount of 50% or more is hydrolyzed by an enzymatic splitting method. When the raw fat and/or oil is hydrolyzed by the enzymatic splitting method, the resulting fatty acids and glycerin are subjected to an esterification reaction using an enzyme, and a deodorization treatment is carried out such that the deodorization time and the deodorization temperature fall within certain ranges, the content of trans-unsaturated fatty acids among the constituent fatty acids of a diacylglycerol-rich fat and/or oil can be reduced, and there is obtained a fat and/or oil that is good in flavor such as umami taste and rich body taste. The term "enzymatic degradation method" refers to a method of obtaining fatty acids and glycerin by adding water to a raw fat and/or oil and allowing the mixture to react under low temperature conditions using an enzyme such a lipase, as a catalyst.

As for the method of hydrolyzing a raw fat and/or oil, an enzymatic splitting method is used, but a high temperature high pressure splitting method may also be used to the extent of not largely decreasing the quality of the resulting final product. Specifically, if the amount of the fatty acids obtainable by hydrolysis is an amount equivalent to less than 30%, and preferably an amount equivalent to less than 10%, of the total amount, a high temperature high pressure splitting method may also be used. In this case, any of the enzymatic splitting method and the high temperature high pressure splitting method may be carried out first, but it is preferable to first carry out the enzymatic splitiing method, from the viewpoint that high splitting ratio can be achieved. Here, the term "high temperature high pressure splitting method" means a method of obtaining fatty acids and glycerin by contacting the raw fat and/or oil with water under high temperature and high pressure conditions in hydrolysis reaction. The hydrolysis based on the high temperature high pressure splitting method can be carried out in a batch mode, a continuous mode or a semi-continuous mode, and the supply of partially hydrolyzed fatty acids and water into the apparatus may be either in a co-current mode or in a counter-current mode.

Preferable as the enzyme used in the enzymatic splitting method is a lipase. As for the lipase, animal-derived and plant-derived lipases, as well as commercially available lipases derived from microorganisms, and immobilized enzymes prepared by immobilizing lipases can be used. Examples of an enzyme for fat and/or oil splitting include lipases derived from microorganisms such as genus Rhizopus, genus Aspergillus, genus Chromobacterium, genus Mucor, genus Pseudomonas, genus Geotrichum, genus Penicillium and genus Candida, and animal lipases such as pancreatic lipases. To obtain a high splitting rate, a lipase having no regiospecificity (random type) is acceptable, and the microbial origin may be the genus Pseudomonas, genus Candida, or the like.

In regard to the hydrolysis based on an enzymatic splitting method of fat and/or oil, it is preferable to use an immobilized enzyme having an enzyme immobilized on a carrier, from the viewpoint of effectively utilizing the enzymatic activity. As for the immobilized enzyme, it is preferable to use a lipase supported on a carrier for immobilization. Examples of the immobilization carrier include inorganic carriers such as celite, diatomaceous earth, kaolinite, silica gel, molecular sieves, porous glass, activated carbon, calcium carbonate, and ceramics; ceramics powder; organic polymers such as polyvinyl alcohol, polypropylene, chitosan, ion-exchanged resins, hydrophobic adsorption resins, chelate resins and synthetic adsorption resins; and the like, and particularly from the viewpoint of having high water-retaining power, ion-exchanged resins are preferable. Among the ion-exchanged resins, porous resins are preferable from the viewpoint of having a large surface area and thereby being capable of increasing the adsorption amount of the enzyme.

The particle size of the resin that is used as the immobilization carrier is preferably 100 to 1000 $\mu$m, and more preferably 250 to 750 $\mu$m. The pore diameter is preferably 10 to 150 nm, and more preferably 10 to 100 nm. The material may be a phenol formaldehyde-based, polystyrene-based, acrylamide-based or divinylbenzene-based resin, and particularly, a phenol formaldehyde-based resin (for example, DUOLITE A-568 manufactured by Rohm and Haas Company) is preferable from the viewpoint of enhancing the enzyme adsorbability.

When an enzyme is immobilized, the enzyme may be directly adsorbed to a carrier. However, in order to render an adsorbed state so as to exhibit high activity, the carrier may be treated in advance with an oil-soluble fatty acid or a derivative thereof before the enzyme adsorption, and then put to use. The oil-soluble fatty acid that is used may be a saturated or unsaturated, linear or branched fatty acid having 8 to 18 carbon atoms, which may be substituted with a hydroxyl group. Specific examples include capric acid, lauric acid, mystyric acid, oleic acid, linolic acid, $\alpha$-linoleic acid, ricinolic acid, isostearic acid, and the like. Derivatives thereof include esters of these fatty acids with monohydric or polyhydric alcohols, phospholipids, and derivatives formed by adding ethylene oxide to these esters. Specific examples thereof include methyl esters, ethyl esters, monoglycerides and diglycerides of the aforementioned fatty acids, ethylene oxide adducts thereof, polyglycerin esters, sorbitan esters, sucrose esters, and the like. These oil-soluble fatty acids or derivatives thereof may also be used in combination of two or more species.

As for the method of contacting these oil-soluble fatty acids or derivatives thereof with a carrier, these substances may be added directly to a carrier that is in water or an organic solvent; however, in order to enhance the dispersibility, a fat-soluble fatty acid or a derivative thereof may be first dispersed and dissolved in an organic solvent, and then the solution may be added to a carrier that has been dispersed in water. This organic solvent may be chloroform, hexane, ethanol, or the like. The amount of use of the fat-soluble fatty acid or a derivative thereof is preferably 1 to 500 parts, and more preferably 10 to 200 parts, relative to 100 parts by weight (hereinafter, simply indicated as "parts") of the carrier. The contact temperature is preferably 273 to 373 K, and more preferably 293 to 333 K, and the contact time is preferably about 5 minutes to 5 hours. The carrier that has been done with this treatment is filtered to be recovered, but may also be dried. The drying temperature is preferably 288 to 373 K, and drying under reduced pressure may also be carried out.

The temperature for carrying out the immobilization of enzyme can be determined based on the characteristics of the enzyme, but the temperature is preferably a temperature at which deactivation of the enzyme does not occur, that is, 273 to 333 K, and more preferably 278 to 313 K. The pH of the enzyme solution that is used at the time of immobilization is acceptable as long as it falls into a range where denaturation of the enzyme does not occur, and can be determined based on the characteristics of the enzyme as in the case of temperature, but pH 3 to 9 is preferable. A buffer solution is used so that the pH can stay at such a level, and examples of the buffer solution include an acetate buffer solution, a phosphate buffer solution, a Tris-hydrochloric acid buffer solution, and the like. The enzyme concentration in the enzyme solution is equal to or lower than the saturation solubility of the enzyme from the viewpoint of immobilization efficiency, and is preferably a sufficient concentration. As for the enzyme solution, a supernatant from which the insoluble has been removed by centrifugation, or a solution that has been purified by ultrafiltration or the like can also be used as necessary. The enzyme weight that is used herein may vary depending on the enzyme activity, but is preferably 5 to 1000 parts, and more preferably 10 to 500 parts, relative to 100 parts of the carrier.

From the viewpoint of bringing the enzyme to a state adequate for the hydrolysis reaction after the immobilization, it is preferable to recover the immobilized enzyme from the enzyme solution by filtration, remove any excess moisture, and then contact the enzyme with the raw fat and/or oil that serves as the reaction substrate, without drying. The moisture content in the immobilized enzyme after the contacting may vary depending on the type of the carrier that is used, but the content is preferably 0.1 to 100 parts, more preferably 1 to 50 parts, and even more preferably 5 to 50 parts, relative to 100 parts of the immobilization carrier. At this time, the immobilized enzyme may be enclosed in a packing vessel such as a column, and the fat and/or oil may be circulated therethrough by a pump or the like, or the immobilized enzyme may also be dispersed in the fat and/or oil. A favorable temperature for contacting is 273 to 333 K, and this temperature can be selected based on the characteristics of the enzyme. Furthermore, a favorable time period for contacting is about one hour to 48 hours, and it is preferable to filter and recover the immobilized enzyme upon completion of this contacting, from the viewpoint of industrial productivity.

The hydrolytic activity of the immobilized enzyme is preferably in the range of 20 U/g or greater, more preferably 100 to 10000 U/g, and even more preferably 500 to 5000 U/g. Here, 1 U of an enzyme represents the splitting ability of the enzyme that produces 1 $\mu$mol of free fatty acids in one minute, when a mixed liquid of fat and/or oil:water = 100:25 (weight ratio) is hydrolyzed for 30 minutes while mixed under stirring at 313 K.

According to the present invention, the hydrolysis based on the enzymatic splitting method of a fat and/or oil can be carried out in a batch mode, a continuous mode or a semi-continuous mode, and the supply of the raw fat and/or oil and water into the apparatus may be either in a co-current mode or a counter-current mode. The raw fat and/or oil that is supplied to the hydrolysis reaction apparatus is preferably subjected to degassing or deoxygenation in advance, from the viewpoint of suppressing oxidation of the fatty acids.

The amount of the immobilized enzyme that is used in the reaction of the enzymatic splitting method can be appropriately determined while taking the activity of the enzyme into consideration, but the amount is preferably 0.01 to 30 parts, more preferably 0.1 to 15 parts, and even more preferably 0.2 to 10 parts, relative to 100 parts of the raw fat and/or oil. The amount of water is preferably 10 to 200 parts, more preferably 20 to 100 parts, and even more preferably 30 to 80 parts, relative to 100 parts of the raw fat and/or oil. The water may be any of distilled water, ion-exchanged water, tap water, well water and the like. The water may also be mixed with other water-soluble components such as glycerin. If necessary, a buffer solution at pH 3 to 9 may also be used so as to maintain stability of the enzyme.

The reaction temperature is preferably set to 273 to 343 K, more preferably 293 to 323 K, which are temperatures that draw out the activity of the enzyme more effectively and do not induce crystallization of the free fatty acids generated by hydrolysis. It is also preferable to carry out the reaction in the presence of an inert gas, so as to avoid the contact with air as far as possible.

Subsequently, the obtained fatty acids and glycerin are esterified to obtain a diacylglycerol-rich fat and/or oil. The method of esterifying fatty acids and glycerin is preferably based on an enzymatic method, from the viewpoint that the method does not increase the content of trans-unsaturated fatty acids in the final fat and/or oil product. As for the enzyme that is used in the esterification reaction, it is preferable to use a lipase, and it is far preferable to use an immobilized enzyme, in view of costs. Examples of the lipase and immobilization carrier include the same ones as described above.

The fat and/or oil with high diacylglycerol content obtained by the esterification reaction can be commercialized after undergoing a post-treatment. In regard to the post-treatments, it is preferable to perform the respective processes of deacidification (removal of unreacted fatty acids), acid treatment, water washing and deodorization. The deacidification process means a process of distilling under reduced pressure the fat and/or oil with high diacylglycerol content obtained by the esterification reaction, to thereby remove fatty acids and monoacylglycerols from the esterification reaction oil. The acid treatment process means a process of adding a chelating agent such as citric acid to the deacidified oil, mixing them, and further dehydrating the mixture under reduced pressure. The resulting acid-treated oil may also be subjected to a decoloration process based on contacting with an adsorbent, from the viewpoint of making the color and flavor more satisfactory. The water washing process means a process of performing an operation of adding water to the acid-treated oil and vigorously stirring the mixture to effect oil-water separation. The water washing is preferably repeated plural times (for example, three times) to obtain a water-washed oil.

The deodorization treatment is basically carried out by steam distillation under reduced pressure, and may be in a batch mode, a semi-continuous mode, a continuous mode, or the like. In the case of treating a small amount, it is preferable to use a batch mode, and in the case of treating a large amount, it is preferable to use a semi-continuous mode or a continuous mode. Examples of the apparatus used to carry out a semi-continuous mode include a Girdler type deodorization apparatus constructed from a deodorization tower equipped with several stages of trays. This apparatus performs deodorization, as the fat and/or oil to be deodorized is supplied from the top, and the oil moves while intermittently descending to the tray of lower stage in sequence. Examples of the apparatus used to carry out a continuous mode include a thin layer deodorization apparatus in which the deodorization tower is packed with a structure that is well balanced between the gas-liquid contact efficiency and a low pressure loss, and thus the efficiency of contact with steam has been enhanced.

The deodorization treatment can be performed by a method of performing the treatment using either the thin layer deodorization apparatus or the tray type deodorization apparatus alone, or a method of performing the treatment by combining the deodorization treatment using a thin layer deodorization apparatus and the deodorization treatment using a tray type deodorization treatment. In the case of the present invention, a method of conducting deodorization using either a thin layer type column or a tray type deodorization apparatus alone, from the viewpoints of the cost of apparatus, the content of trans-unsaturated fatty acids, and the flavor characteristic to diacylglycerols.

Through the deodorization treatment, the odorous components contained in the water-washed oil are removed, carotenoid-based coloring matters become light-colored as they are thermally degraded, and those impurity materials contained in trace amounts are inactivated to become stable materials. Therefore, in the deodorization using a conventional fat and/or oil, a fat and/or oil preferable in terms of flavor is obtained by establishing the conditions more sufficiently . However, in regard to a diacylglycerol-rich fat and/or oil, since the rich body taste is affected by the deodorization process, the quality of the product depends on the conditions for the deodorization treatment.

According to the present invention, this deodorization treatment is carried out such that the deodorization time (x) and the deodorization temperature (y) fall in the ranges that satisfy the following conditions (i) and (ii).

$$\text{(i)} \quad y \geq -2.59x + 566$$

$$\text{(ii)} \quad y \leq 617x^{-0.0455} \quad (\text{provided that } 475 \leq y \leq 563)$$

If the deodorization treatment is carried out at a temperature lower than the range defined by the condition (i), or if the deodorization treatment is carried out at a deodorization temperature (y) lower than 475 K, a fat and/or oil reduced in the sensation of stimulation and heaviness may not be obtained. If the deodorization treatment is carried out for a time longer than the range defined by the condition(ii), a satisfactory rich body taste may not be obtained. Here, x represents the deodorization time (minutes) and y represents the deodorization temperature (K). However, if the temperature changes over time during the deodorization process, the average value of the changing temperatures is taken. Furthermore, it is preferable to carry out the deodorization treatment to the extent that the following conditions (iii) and (iv):

$$\text{(iii)} \quad y \geq -2.29x + 566$$

$$\text{(ii)} \quad y \leq (617x^{-0.0455}) - 3 \quad (\text{provided that } 480 \leq y \leq 558)$$

are satisfied, from the viewpoint of enhancing the deodorization efficiency and the flavor, and it is even more preferable that the deodorization temperature (y) be 485 K or higher and 553 K or lower.

In the present invention, since the deodorization treatment is carried out under the foregoing conditions (i) and (ii), the deodorization time changes with the deodorization treatment temperature. Specifically, in the case of performing the deodorization treatment at 475 to 480 K, it is preferable to set the deodorization time at from 35 to 315 minutes; in the case of performing the deodorization treatment at 480 to 490 K, to set the deodorization time at from 35 to 210 minutes; in the case of performing the deodorization treatment at 490 to 500 K, to set the deodorization time at from 30 to 150 minutes; in the case of performing the deodorization treatment at 500 to 510 K, to set the deodorization time at from 20 to 90 minutes; in the case of performing the deodorization treatment at 510 to 520 K, to set the deodorization time at from 15 to 52 minutes; in the case of performing the deodorization treatment at 520 to 530 K, to set the deodorization time at from 12 to 45 minutes; in the case of performing the deodorization treatment at 530 to 540 K, to set the deodorization time at from 9 to 30 minutes; in the case of performing the deodorization treatment at 540 to 550 K, to set the deodorization time from at 6 to 22 minutes; and in the case of performing the deodorization treatment at 550 to 563 K, to set the deodorization time at from 3 to 15 minutes.

The deodorization treatment is preferably steam distillation under reduced pressure, and it is preferable to set the amount of steam used at from 0.3 to 20%, and even more preferably from 0.5 to 10%, based on the fat and/or oil, from the viewpoint of improving the flavor characteristic to diacylglycerols, such as the "umami taste" and "rich body taste". It is also preferable to set the pressure at from 0.01 to 4 kPa, and even more preferably from 0.06 to 0.6 kPa, from similar viewpoints.

Among others, it is preferable to set the deodorization temperature at from 528 to 558 K, and to set the amount of steam at from 0.3 to 3%, more preferably from 0.4 to 2.5%, and even more preferably from 0.5 to 2.2%, based on the fat and/or oil, from the viewpoint of improving the flavor characteristic to diacylglycerols, such as the "umami taste" and "rich body taste". It is more preferable to set the deodorization temperature at from 523 to 528 K, and to set the amount of steam at from 2.1 to 5%, more preferably from 2.2 to 4.5%, and even more preferably from 2.5 to 4%, based on the fat and/or oil, from similar viewpoints. It is even more preferable to set the deodorization temperature at from 480 to 523 K, and to set the amount of steam at from 2.1 to 10%, more preferably from 2.2 to 8%, and even more preferably from 2.5 to 6%, based on the fat and/or oil, from similar viewpoints.

The time for temperature elevation to the deodorization temperature is preferably set at from 0.5 to 60 minutes to heat from the temperature of 343 K to the temperature of 473 K, and at from 0.5 to 45 minutes to heat from the temperature of 473 K to the deodorization temperature, in view of the content of trans-unsaturated fatty acids. The time for temperature elevation is more preferably set at from 1 to 30 minutes to heat from the temperature of 343 K to the temperature of 473 K, and at from 1 to 20 minutes to heat from the temperature of 473 K to the deodorization temperature; and is even more preferably set at from 2 to 20 minutes to heat from the temperature of 343 K to the temperature of 473 K, and at from 2 to 15 minutes to heat from the temperature of 473 K to the deodorization temperature. The time for cooling from the deodorization temperature is preferably set at from 0.2 to 35 minutes to cool from the deodorization temperature to the temperature of 473 K, and at from 0.2 to 40 minutes to cool from the temperature of 473 K to 343 K, in view of the content of trans-unsaturated fatty acids. The time for cooling is more preferably set at from 0.5 to 25 minutes to cool from the deodorization temperature to the temperature of 473 K, and at from 0.5 to 30 minutes to cool from the temperature of 473 K to 343 K; and is even more preferably set at from 1 to 20 minutes to cool from the deodorization temperature to the temperature of 473 K, and at from 1 to 25 minutes to cool from the temperature of 473 K to 343 K.

As a result of the deodorization treatment, the increment in the amount of trans-unsaturated fatty acids in the purification processes can be suppressed to 1% or less, and a diacylglycerol-rich fat and/or oil in which the content of trans-unsaturated fatty acids among the total fatty acids constituting the fat and/or oil is as low as 2% or less, can be obtained. The content of trans-unsaturated fatty acids in the fat and/or oil with high diacylglycerol content is more preferably 0 to 1.5%, and even more preferably 0.1 to 1.2%, from the viewpoint of physiological effects.

The content of diacylglycerols in the fat and/or oil with high diacylglycerol content is preferably 50% or greater, more preferably 60 to 99%, even more preferably 70 to 98%, and even more preferably 80 to 95%, from the viewpoint of physiological effects.

The diacylglycerols are preferably such that 80 to 100%, more preferably 90 to 100%, even more preferably 93 to 99%, and far more preferably 94 to 99%, of the constituent fatty acids are unsaturated fatty acids (UFA), from the viewpoints of the external appearance, physiological effects, and industrial productivity of the fat and/or oil. Here, the number of carbon atoms of these unsaturated fatty acids is preferably 14 to 24, and more preferably 16 to 22. Conventionally, a fat and/or oil with high unsaturated fatty acid content is likely to turn into trans-unsaturated fatty acids under heating; however, according to the method of the present invention, the generation of trans-unsaturated fatty acids can be suppressed low even in the production of a fat and/or oil with a high content of unsaturated fatty acids as such.

Furthermore, among the unsaturated fatty acids, the content of oleic acid is preferably 20 to 90%, more preferably 25 to 80%, and even more preferably 30 to 70%; the content of linolic acid is preferably 5 to 65%, more preferably 10 to 60%, and even more preferably 15 to 55%; and the content of linolenic acid is preferably less than 15%, more preferably 0 to 13%, and even more preferably 1 to 12%.

The fat and/or oil with high diacylglycerol content produced by the method of the present invention has good flavor and good color. Furthermore, since the fat and/or oil does not produce trans-unsaturated fatty acids and has a high content of diacylglycerols, it is useful as a fat and/or oil having high physiological effects.

EXAMPLES

[Analysis methods]

(i) Content of diacylglycerols

About 10 mg of a sample and 0.5 mL of trimethylsilylating agent ("SILYLATING AGENT TH", manufactured by Kanto Chemical Co., Inc.) were added to a glass sample bottle, and the bottle was stoppered tightly and heated for 15 minutes at 343 K. To this bottle, 1.5 mL of water and 1.5 mL of hexane were added, and the mixture was shaken. The mixture was left to stand still, and then the upper layer was subjected to gas chromatography (GLC), to perform an analysis of the glyceride composition.

(ii) Content of trans-unsaturated fatty acids among constituent fatty acids

This content refers to the value obtained by preparing fatty acid methyl esters according to the "Method for Preparing Fatty Acid Methyl Esters (2.4.1.2-1996)" in the "Standard Methods for the Analysis of Fats, Oils and Related Materials," edited by Japan Oil Chemists' Society, and measuring the resulting sample according to the American Oil Chemists' Society Official Method Ce 1f-96 (GLC method).

(iii) Color

The color of a deodorized oil refers to the value measured by the American Oil Chemists' Society Official Method Ca 13e-92 (Lovibond method) using a 5.25-inch cell, and determined by the following expression (1):

$$\text{Color C} = 10R + Y \qquad (1)$$

wherein R = Red value, and Y = Yellow value)

[Method for producing immobilized enzyme for hydrolysis]

500 g of DUOLITE A-568 (manufactured by Rohm & Haas Company) was stirred for one hour in 5000 mL of a 0.1 N aqueous solution of sodium hydroxide. Subsequently, the carrier was washed for one hour with 5000 mL of distilled water, and the carrier was subjected to pH equilibration for 2 hours, using 5000 mL of a 500 mM phosphate buffer solution (pH 7). Subsequently, the carrier was subjected to pH equilibration two times for 2 hours each, using 5000 mL of a 50 mM phosphate buffer solution (pH 7). After this, the carrier was recovered by performing filtration, and then ethanol substitution was carried out for 30 minutes using 2500 mL of ethanol. After filtering the carrier, 2500 mL of ethanol containing 500 g of soybean fatty acids was added thereto, and the soybean fatty acids were adsorbed to the carrier for 30 minutes. Then, after recovering the carrier by filtration, the carrier was washed four times with 2500 mL of a 50 mM phosphate buffer solution (pH 7), and ethanol was removed. The carrier was recovered by filtration. Subsequently, the carrier was contacted with 10000 mL of a 10% solution of a commercially available lipase that acts on a fat and/or oil (lipase AY "AMANO", manufactured by Amano Enzyme, Inc.) for 4 hours, to effect immobilization. Furthermore, the immobilized enzyme was recovered by filtration and was washed with 2500 mL of a 50 mM acetate buffer solution (pH 7), to remove any unimmobilized enzyme or proteins. The operations described above were all carried out at 293 K. The ratio of immobilization was determined from the difference between the residual activity of the enzyme solution after immobilization and the activity of the enzyme solution before immobilization, and the ratio was found to be 95%. Subsequently, 2000 g of deodorized soybean oil was added thereto, and the mixture was stirred for 2 hours at 313 K. Then, the immobilized enzyme was filtered to separate from the deodorized soybean oil, and was used as an immobilized enzyme. The immobilized enzyme thus obtained was washed three times with a raw material oil, which was the actual substrate used in the reaction, and was filtered, before use. The hydrolytic activity was measured, and was found to be 2500 U/g.

Examples 1 to 10

[Raw fat and/or oil]

Each of the fats and/or oils indicated in Table 2 was used as a raw fat and/or oil. The measurement of the content of trans-unsaturated fatty acids in the raw fat and/or oil was carried out according to the method described above. The fatty acid compositions of the raw fat and/or oil are presented in Table 1.

[Table 1]

|  |  | Undeodorized rapeseed oil | Deodorized rapeseed oil | Undeodorized soybean oil |
|---|---|---|---|---|
| Glyceride composition (wt%) | TAG | 98.6 | 98.7 | 98.9 |
|  | DAG | 1.3 | 1.3 | 1.0 |
|  | MAG | 0.0 | 0.0 | 0.0 |
|  | FFA | 0.1 | 0.0 | 0.1 |
| Fatty acid composition (wt%) | C16:0 | 4.1 | 4.0 | 10.4 |
|  | C18:0 | 1.8 | 1.9 | 4.4 |
|  | C18:1 | 59.0 | 59.2 | 24.2 |
|  | C18:2 | 19.9 | 19.9 | 52.3 |
|  | C18:3 | 11.3 | 11.1 | 6.9 |
|  | (Trans-unsaturated fatty acids) | (0.1) | (1.7) | (0.0) |
|  | Others | 3.9 | 3.9 | 1.8 |

[Hydrolysis based on enzymatic splitting method]

The raw fat and/or oil indicated in Table 2 were subjected to a hydrolysis based on an enzymatic splitting method using the immobilized enzyme for hydrolysis as described above. A 10-L four-necked glass flask equipped with a 125-mm crescent blade impeller, was charged with 400 g (on a dry basis) of the immobilized enzyme for hydrolysis. The weight on a dry basis of the immobilized enzyme was determined by treating the immobilized enzyme of the same batch as that was in use, to remove any oil components attached to the immobilized enzyme using acetone and hexane, and further dehydrating the resulting enzyme under reduced pressure.

4000 g of a raw fat and/or oil was introduced to a 10-L four-necked glass flask, and then the fat and/or oil was heated to 313 K, while stirred at 200 r/min. Subsequently, 2400 g of distilled water warmed to 313 K was introduced thereto, and thus a hydrolysis reaction was carried out. During this time, the inside of the 10-L four-necked glass flask was maintained under a nitrogen atmosphere.

After 20 hours from the initiation of reaction, the immobilized enzyme for hydrolysis was separated from the reaction liquid by filtration, and the reaction liquid was centrifuged at a speed of rotation of 5000 r/min for 10 minutes, to thereby remove the aqueous layer. Thus, fatty acids were obtained. The fatty acids were further dehydrated under reduced pressure at a temperature of 343 K and at a degree of vacuum of 400 Pa for 30 minutes. The contents of trans-unsaturated fatty acids of the fatty acids obtained by degrading the raw fat and/or oil, or of the fatty acids obtained after blending a plurality of fatty acids, are presented in Table 2.

[Esterification by enzymatic method]

172 g (an amount equivalent to 5% of the total amounts of fatty acids and glycerin) of an immobilized lipase (LIPOZYME RM IM manufactured by Novozymes, co. jp) was introduced into a 5-L four-necked glass flask. Subsequently, 3000 g of the fatty acids obtained by enzymatically degrading the raw fat and/or oil indicated in Table 2, was adjusted to a temperature of 323 K and then was introduced into the flask. While the content of the flask was stirred at 500 r/min, at a temperature of 323 K, 446 g of glycerin was introduced into the flask such that the molar ratio of fatty acids and glycerin would be 2:1, and thus the reaction was initiated. One minute after the initiation of reaction, pressure reduction was initiated, and the esterification reaction was carried out at a degree of vacuum of 400 Pa for 3 hours. After the reaction, the immobilized enzyme was separated by filtration, and thus an esterification reaction oil was obtained.

[Deacidification treatment]

The esterification reaction oil was distilled using a wiped film evaporating apparatus (Shinko Pantech Co., Ltd.,

Model 2-03, inner diameter 5 cm, area of heat transfer 0.03 m$^2$), under the operation conditions of a heater temperature of 508 K, a pressure of 3.3 Pa, and a flow amount of 150 ml/min. Thus, a deacidified oil was obtained.

[Acid treatment]

A 10% aqueous solution of citric acid was added to the deacidified oil in an amount of 2%, and the mixture was mixed at 400 r/min, at a temperature of 343 K for 30 minutes. Subsequently, while the mixture was mixed at 400 r/min, at a temperature of 373 K and a degree of vacuum of 400 Pa, the mixture was dehydrated under reduced pressure for 30 minutes. Thus, an acid-treated oil was obtained.

[Water washing treatment]

Distilled water warmed to a temperature of 373 K was added to the acid-treated oil in an amount of 10%, and the mixture was vigorously mixed at 600 r/min, at a temperature of 373 K for 30 minutes. Then, the mixture was centrifuged to separate the oil phase. This water washing operation was carried out three times, and the oil phase was dehydrated under reduced pressure at a temperature of 373 K and a degree of vacuum of 400 Pa for 30 minutes, to obtain a water-washed oil. In Examples 9 and 10, a decoloration treatment as described below was carried out after the acid treatment, and then the water washing treatment was carried out.

[Decoloration treatment]

In Examples 9 and 10, after the acid treatment had been carried out, 2% of activated white clay and 0.5% of activated coal were added to the acid-treated oil. While the mixture was mixed at 400 r/min at a temperature of 383 K and a degree of vacuum of 400 Pa, the acid-treated oil was subjected to decoloration under reduced pressure for 30 minutes. The mixture was cooled to a temperature of 373 K or lower, and then the adsorbents were separated by filtration. The resulting decolored oil was subjected to the water washing treatment, to obtain a water-washed oil.

[Deodorization treatment]

The deodorization treatment was performed in a batch mode. The vacuum pump used was a rotary vacuum pump, TYPE 160VP-D CuteVac, manufactured by Hitachi, Ltd. 100 g of the water-washed oil was introduced into a 300-mL glass Claisen's flask, and then a steam generating apparatus was connected to the 300-mL glass Claisen's flask through a capillary glass tube having an inner diameter of 2.5 mm. Nitrogen was passed through the content by bubbling at a flow rate of 1 L/min, at a temperature of 343 K for 10 minutes, and thus the apparatus was completely purged with nitrogen. After it was confirmed that there was no vacuum leak, the inside of the flask was brought to a vacuum state with the vacuum pump, and the system was heated with a mantle heater. A heating time of 6 to 8 minutes to heat from the temperature of 343 K to the temperature of 473 K, and 2 to 5 minutes to heat from the temperature of 473 K to the deodorization temperature, was required. The deodorization temperature, deodorization time and amount of steam were set at the respective conditions indicated in Table 2, and the pressure was set at 0.2 to 0.4 kPa. After completion of the deodorization, the mantle heater was removed, and the system was cooled with a cold air blower, over 1 to 2 minutes to cool from the deodorization temperature to the temperature of 473 K, and 5 to 7 minutes to cool from the temperature of 473 K to the temperature of 343 K. After the system had been cooled to the temperature of 343 K, nitrogen was blown into the deodorization apparatus to return the system to normal pressure. Tocopherol was added in an amount of 200 ppm based on the water-washed oil, to obtain a deodorized oil.

[0055]

[Table 2]

| | | | Example | | | | | | | | | |
|---|---|---|---|---|---|---|---|---|---|---|---|---|
| | | | 1 | 2 | 3 | 4 | 5 | 6 | 7 | 8 | 9 | 10 |
| Raw fat and/or oil | Rapeseed oil | Purification | Deodorized | Undeodorized | Undeodorized | Undeodorized | Undeodorized | Undeodorized | Undeodorized | Undeodorized | Undeodorized | Undeodorized |
| | | Hydrolysis method | Enzymatic | Enzymatic | Enzymatic | Enzymatic | Enzymatic | Enzymatic | Enzymatic | Enzymatic | Enzymatic | Enzymatic |
| | | Amount of use (wt%) | 30 | 30 | 100 | 100 | 100 | 40 | 40 | 40 | 100 | 100 |
| | Soybean oil | Purification | Undeodorized | Undeodorized | | | | Undeodorized | Undeodorized | Undeodorized | | |
| | | Hydrolysis method | Enzymatic | Enzymatic | | | | Enzymatic | Enzymatic | Enzymatic | | |
| | | Amount of use (wt%) | 70 | 70 | 0 | 0 | 0 | 60 | 60 | 60 | 0 | 0 |
| | Trans-unsaturated fatty acid content after hydrolysis (wt%) | | 0.5 | 0.0 | 0.0 | 0.0 | 0.0 | 0.0 | 0.0 | 0.0 | 0.0 | 0.0 |
| Esterification reaction oil | Presence or absence of decoloration treatment | | Absence | Absence | Absence | Absence | Absence | Absence | Absence | Absence | Presence | Presence |
| | Deodorization conditions | Temperature (K) | 508 | 508 | 518 | 493 | 483 | 493 | 508 | 533 | 493 | 483 |
| | | Time (minutes) | 60 | 60 | 34 | 60 | 60 | 120 | 30 | 20 | 60 | 60 |
| | | Amount of steam (wt%) | 3 | 3 | 2 | 6 | 8 | 9 | 2 | 2 | 4 | 5 |
| | Color (10R+Y) | | 19.8 | 20 | 16 | 19 | 22 | 19 | 21 | 18 | 9 | 11 |
| | DAG content (wt%) | | 86 | 88 | 86 | 88 | 89 | 94 | 94 | 94 | 88 | 89 |
| | Trans acid content (wt%) | | 1.0 | 0.8 | 0.8 | 0.3 | 0.2 | 0.5 | 0.3 | 1.0 | 0.4 | 0.2 |
| | Flavor evaluation | Rich body taste | 5 | 5 | 5 | 5 | 5 | 4 | 4 | 4 | 4 | 4 |
| | | Sensation of stimulation, heaviness | 5 | 5 | 4 | 3 | 3 | 4 | 3 | 3 | 5 | 4 |

EP 2 175 030 B1

[Sensory evaluation]

The evaluation of flavor (rich body taste, sensation of stimulation and heaviness) was performed by a panel of five members. Each member ate 0.5 to 5 g of raw fat and/or oil, and performed a sensory evaluation according to the criteria indicated in the following Table 3. The results are presented in Table 2.

The term "rich body taste" as used herein refers to a preferable flavor that is characteristic to the diacylglycerols produced by the production method of the present invention, and to a flavor that causes good taste or the like to spread in the mouth and has richness well-balanced with palatability. Furthermore, the terms "sensation of stimulation" and "heaviness" refer to a flavor attributable to an undeodorized fat and/or oil that is a raw material, or an undesirable flavor coming from the impurities produced in the process for production of a diacylglycerol-rich fat and/or oil, and to the stimulating sensation occurring in the mouth or the throat (sensation of stimulation) and the sensation in the mouth that feels like sticking viscously (heaviness).

[Table 3]

| [Criteria for evaluation of flavor] | | |
|---|---|---|
| | Rich body taste | Sensation of stimulation, heaviness |
| 5 | Rich body taste is very good | Sensation of stimulation and heaviness are absent, and the sensation is very good |
| 4 | Rich body taste is good | Sensation of stimulation and heaviness are absent, and the sensation is good |
| 3 | Rich body taste is slightly present | Sensation of stimulation and heaviness are absent |
| 2 | Rich body taste is short | Sensation and/or heaviness is slightly present |
| 1 | Rich body taste lacks | Sensation and/or heaviness is present |

Comparative Examples 1 to 13

[Hydrolysis based on enzymatic splitting method]

The raw fat and/or oil indicated in Table 1 were subjected to a hydrolysis based on an enzymatic splitting method using an immobilized enzyme for hydrolysis, by the same operations as those of Example 1 or the like. Only in Comparative Example 13, hydrolysis was carried out based on an enzymatic splitting method using an enzyme (lipase AY "AMANO", manufactured by Amano Enzyme, Inc.) that was not immobilized on a carrier.

[Hydrolysis based on high temperature high pressure splitting method]

Using a high pressure hot water type splitting apparatus of oil-water counter-current type, the raw fat and/or oil was continuously transported from the lower side of the apparatus, and water was continuously transported from the upper side of the apparatus. The amount of liquid transport was 50 parts of water with respect to 100 parts of the raw fat and/or oil. In this case, the average retention time (hr) in the splitting tower (tower volume ($m^3$)/(flow rate of raw oil ($m^3$/hr) + flow rate of water ($m^3$/hr)) was about 4 hr. Inside the apparatus, the raw fat and/or oil was heated by high pressure hot water (5.0 MPa, 513 K). The reaction liquid was appropriately collected from a sampling port present in the upper part of the high pressure hot water type hydrolysis apparatus of oil-water counter-current type, and the sampled reaction liquid was cooled to 298 K in a nitrogen-sealed, light-blocked state. Thereafter, the reaction liquid was centrifuged (5,000g, 30 minutes) to remove the aqueous layer, and then the fatty acid layer was dehydrated under reduced pressure at a temperature of 343 K and a degree of vacuum of 400 Pa for 30 minutes, to obtain fatty acids.

[Esterification reaction, etc.]

The respective treatments of esterification reaction, deacidification, acid treatment, water washing and decoloration were carried out by the same operations as those of Example 1 or the like. Only in Comparative Example 5, a decoloration treatment was carried out by the same operation as that of Example 9 or 10.

[Deodorization treatment]

The same operation as that of Example 1 or the like was carried out, except that the deodorization treatment was carried out under the conditions indicated in Table 4. The results are presented in Table 4.

[0062]

[Table 4]

| | | | Comparative Example | | | | | | | | | | | | |
|---|---|---|---|---|---|---|---|---|---|---|---|---|---|---|---|
| | | | 1 | 2 | 3 | 4 | 5 | 6 | 7 | 8 | 9 | 10 | 11 | 12 | 13 |
| Raw fat and/or oil [material] | Rapeseed oil | Purification | Deodorized | Undeodorized | Deodorized | Undeodorized | Undeodorized | Undeodorized | Undeodorized | Undeodorized | Undeodorized | Undeodorized | Undeodorized | Undeodorized | Undeodorized |
| | | Hydrolysis method | Enzymatic | Enzymatic | Enzymatic | Enzymatic | Enzymatic | Enzymatic | Enzymatic | Enzymatic | Enzymatic | Enzymatic | Enzymatic | Enzymatic | Enzymatic |
| | | Amount of use (wt%) | 30 | 30 | 100 | 100 | 100 | 40 | 40 | 40 | 40 | 40 | 40 | 40 | 100 |
| | Soybean oil | Purification | Undeodorized | Undeodorized | | | | Undeodorized | Undeodorized | Undeodorized | Undeodorized | Undeodorized | Undeodorized | Undeodorized | |
| | | Hydrolysis method | High pressure | High pressure | | | | Enzymatic | Enzymatic | Enzymatic | Enzymatic | Enzymatic | Enzymatic | Enzymatic | |
| | | Amount of use (wt%) | 70 | 70 | 0 | 0 | 0 | 60 | 60 | 60 | 60 | 60 | 60 | 60 | |
| | Trans-unsaturated fatty acid content after hydrolysis (wt%) | | 2.5 | 1.9 | 1.8 | 0.0 | 0.0 | 0.0 | 0.0 | 0.0 | 0.0 | 0.0 | 0.0 | 0.0 | 0.2 |
| | Presence or absence of decoloration treatment | | Absence | Absence | Absence | Absence | Presence | Absence | Absence | Absence | Absence | Absence | Absence | Absence | Absence |
| Esterification reaction oil | Deodorization conditions | Temperature (K) | 518 | 518 | 518 | 473 | 473 | 483 | 493 | 493 | 508 | 508 | 533 | 533 | 513 |
| | | Time (minutes) | 34 | 34 | 34 | 120 | 120 | 30 | 20 | 180 | 10 | 120 | 5 | 40 | 60 |
| | | Amount of steam (wt%) | 2 | 2 | 2 | 10 | 6 | 4 | 5 | 9 | 1.5 | 7 | 0.5 | 4 | 3 |
| | Color (10R+Y) | | 13 | 12 | 18 | 25 | 10 | 40 | 38 | 18 | 31 | 18 | 21 | 17 | 35 |
| | DAG content (wt%) | | 94 | 92 | 86 | 90 | 89 | 94 | 94 | 94 | 94 | 93 | 94 | 93 | 84 |
| | Trans acid content (wt%) | | 3.1 | 2.5 | 2.5 | 0.1 | 0.2 | 0.1 | 0.1 | 0.7 | 0.1 | 1.2 | 0.4 | 2.0 | 1.4 |
| | Flavor evaluation | Rich body taste | 2 | 2 | 1 | 4 | 4 | 4 | 4 | 1 | 5 | 1 | 5 | 1 | 2 |
| | | Sensation of stimulation, heaviness | 4 | 4 | 4 | 0 | 2 | 0 | 1 | 4 | 1 | 4 | 1 | 4 | 4 |

Method for hydrolysis of raw fat and/or oil
Enzymatic: hydrolysis based on enzymatic degradation method
High pressure: hydrolysis based on high temperature high pressure degradation method
DAG: Diacylglycerols

From the results obtained above, it was found that when a fat and/or oil containing undeodorized fat and/or oil in an amount of 50% or more is used as the raw fat and/or oil; the fatty acids and glycerin obtainable by an enzymatic splitting method are subjected to an esterification reaction using an enzyme; and a deodorization treatment is carried out such that the deodorization time and the deodorization temperature fall in certain ranges, a diacylglycerol-rich fat and/or oil that has a low content of trans-unsaturated fatty acids and has a good color and a good flavor characteristic to diacylglycerols, such as "umami taste" and "rich body taste", may be efficiently obtained.

On the other hand, it was found that a fat and/or oil obtained using 100% of a deodorized fat and/or oil as the raw material, has a good color, but has a high content of trans-unsaturated fatty acids among the constituent fatty acids, and feels like having a sensation of stimulation and heaviness. It was also found that an fat and/or oil obtained using the fatty acids and glycerin obtained by hydrolyzing the raw fat and/or oil by a high temperature high pressure splitting method, has no sensation of stimulation and heaviness, but has a high content of trans-unsaturated fatty acids among the constituent fatty acids, and that a fat and/or oil having a satisfactory rich body taste may not be obtained.

**Claims**

1. A method for producing a diacylglycerol-rich fat and/or oil, comprising hydrolyzing a raw fat and/or oil by an enzymatic splitting method to obtain a fatty acid, and then esterifying the fatty acid and a glycerin, wherein the raw fat and/or oil containing an undeodorized fat and/or oil in an amount of 50% by weight or more is used, and a deodorization treatment, which is an operation of removing any odorous components by performing a reduced pressure steam distillation under heating, is carried out after the esterification reaction, to the extent that the relationship between the deodorization time (x) and the deodorization temperature (y) fall in the range defined by the following (i) and (ii):

$$\text{(i) } y >= -2.59x + 566$$

$$\text{(ii) } y <= 617x^{-0.0455},$$

provided that $475 <= y <= 563$,
wherein x represents the deodorization time (minutes), and y represents the deodorization temperature (K), and wherein the deodorization treatment is carried out at a pressure ranging from 0.01 to 4 kPa.

2. The method according to claim 1, wherein the content of trans-unsaturated fatty acids among the constituent fatty acids of the raw fat and/or oil supplied to the hydrolysis reaction, is 1.5% by weight or less.

3. The method according to claim 1 or 2, wherein the esterification reaction of the fatty acid and glycerin is carried out by an enzymatic method.

4. The method according to any one of claims 1 to 3, wherein a diacylglycerol-rich fat and/or oil having a trans-unsaturated fatty acid content among the constituent fatty acids of the fat and/or oil of 2% by weight or less is obtained.

5. The method according to any one of claims 1 to 4, wherein the fat and/or oil with high diacylglycerol content is a fat and/or oil containing diacylglycerols in an amount of 50% by weight or more.

6. The method of claim 1, wherein the deodorization treatment is carried out such that in the case of performing the deodorization treatment at 475 to 480 K, the deodorization time is set in the range of 35 to 315 minutes; in the case of performing the deodorization treatment at 480 to 490 K, the deodorization time is set in the range of 35 to 210 minutes; in the case of performing the deodorization treatment at 490 to 500 K, the deodorization time is set in the range of 30 to 150 minutes; in the case of performing the deodorization treatment at 500 to 510 K, the deodorization time is set in the range of 20 to 90 minutes; in the case of performing the deodorization treatment at 510 to 520 K, the deodorization time is set in the range of 15 to 52 minutes; in the case of performing the deodorization treatment at 520 to 530 K, the deodorization time is set in the range of 12 to 45 minutes; in the case of performing the deodorization treatment at 530 to 540 K, the deodorization time is set in the range of 9 to 30 minutes; in the case of performing the deodorization treatment at 540 to 550 K, the deodorization time is set in the range of 6 to 22 minutes; and in the case of performing the deodorization treatment at 550 to 563 K, the deodorization time is set in the range of 3 to 15 minutes.

**Patentansprüche**

1. Verfahren zur Herstellung von Diacylglycerin-reichem Fett und/oder Öl, umfassend das Hydrolysieren eines Rohfettes und/oder Rohöls durch ein enzymatisches Spaltverfahren, um eine Fettsäure zu erhalten, und dann das Verestern von der Fettsäure und Glycerin, wobei ein Rohfett und/oder Rohöl, das ein nicht-desodoriertes Fett und/oder Öl in einer Menge von 50 Gew.-% oder mehr enthält, verwendet wird, und wobei eine Desodorierungsbehandlung, welche ein Vorgang zum Entfernen der Geruchsbestandteile mittels Durchführen einer Dampfdestillation bei vermindertem Druck unter Erhitzen nach der Veresterungsreaktion ist, soweit durchgeführt wird, dass die Beziehung zwischen der Desodorierungszeit (x) und der Desodorisierungstemperatur (y) in den durch die folgenden (i) und (ii) definierten Bereich fallen:

$$\text{(i)} \quad y \geq -2{,}59x + 566$$

$$\text{(ii)} \quad y \leq 617x^{-0{,}0455},$$

vorausgesetzt, dass $475 \leq y \leq 563$ ist,
wobei x für die Desodorierungszeit (Minuten) steht und y für die Desodorisierungstemperatur (K) steht, und wobei die Desodorierungsbehandlung bei einem Druck im Bereich von 0,01 bis 4 kPa durchgeführt wird.

2. Verfahren gemäß Anspruch 1, wobei der Gehalt an trans-ungesättigten Fettsäuren unter den Fettsäurebestanteilen des Rohfetts und/oder Rohöls, die der Hydrolysereaktion zugeführt werden, 1,5 Gew.-% oder weniger beträgt.

3. Verfahren gemäß Anspruch 1 oder 2, wobei die Veresterungsreaktion von der Fettsäure und Glycerin über ein enzymatisches Verfahren durchgeführt wird.

4. Verfahren gemäß einem der Ansprüche 1 bis 3, wobei ein Diacylglycerin-reiches Fett und/oder Öl mit einem Gehalt an trans-ungesättigten Fettsäuren unter den Fettsäurebestandteilen des Fetts und/oder Öls von 2 Gew.-% weniger erhalten wird.

5. Verfahren gemäß einem der Ansprüche 1 bis 4, wobei das Fett und/oder Öl mit hohem Diacylglyceringehalt ein Fett und/oder Öl ist, das Diacylglycerine in einer Menge von 50 Gew.-% oder mehr enthält.

6. Verfahren gemäß Anspruch 1, wobei die Desodorierungsbehandlung derart durchgeführt wird, dass im Falle der Durchführung der Desodorierungsbehandlung bei 475 bis 480 K die Desodorierungszeit im Bereich von 35 bis 315 Minuten liegt; im Falle der Durchführung der Desodorierungsbehandlung bei 480 bis 490 K die Desodorierungszeit im Bereich von 35 bis 210 Minuten liegt; im Falle der Durchführung der Desodorierungsbehandlung bei 490 bis 500 K die Desodorierungszeit im Bereich von 30 bis 150 Minuten liegt; im Falle der Durchführung der Desodorierungsbehandlung bei 500 bis 510 K die Desodorierungszeit im Bereich von 20 bis 90 Minuten liegt; im Falle der Durchführung der Desodorierungsbehandlung bei 510 bis 520 K die Desodorierungszeit im Bereich von 15 bis 52 Minuten liegt; im Falle der Durchführung der Desodorierungsbehandlung bei 520 bis 530 K die Desodorierungszeit im Bereich von 12 bis 45 Minuten liegt; im Falle der Durchführung der Desodorierungsbehandlung bei 530 bis 540 K die Desodorierungszeit im Bereich von 9 bis 30 Minuten liegt; im Falle der Durchführung der Desodorierungsbehandlung bei 540 bis 550 K die Desodorierungszeit im Bereich von 6 bis 22 Minuten liegt; und im Falle der Durchführung der Desodorierungsbehandlung bei 550 bis 563 K die Desodorierungszeit im Bereich von 3 bis 15 Minuten liegt.

**Revendications**

1. Procédé de production d'une graisse et/ou huile riche en diacylglycérol, comprenant l'hydrolyse d'une graisse et/ou huile brute par un procédé de clivage enzymatique pour obtenir un acide gras, et ensuite l'estérification de l'acide gras et d'une glycérine, dans lequel la graisse et/ou huile brute contenant une graisse et/ou huile non désodorisée en une quantité de 50 % en poids ou plus et/ou sont utilisée(s), et un traitement de désodorisation, qui est une opération d'élimination de quelconques composants odorants par la mise en oeuvre d'une distillation à la vapeur d'eau sous pression réduite en chauffant, est effectué après la réaction d'estérification, dans la mesure où la relation entre le temps de désodorisation (x) et la température de désodorisation (y) tombe dans la plage définie par les (i)

et (ii) suivants :

$$\text{(i)} \quad y >= -2{,}59x + 566$$

$$\text{(ii)} \quad y <= 617x^{-0{,}0455},$$

à condition que 475 <= y <= 563,
dans lequel x représente le temps de désodorisation (minutes), et y représente la température de désodorisation (K), et
dans lequel le traitement de désodorisation est effectué à une pression allant de 0,01 à 4 kPa.

**2.** Procédé selon la revendication 1, dans lequel la teneur en acides gras trans-insaturés parmi les acides gras constitutifs de la graisse et/ou huile brute fournie à la réaction d'hydrolyse est de 1,5 % en poids ou moins.

**3.** Procédé selon la revendication 1 ou 2, dans lequel la réaction d'estérification de l'acide gras et de la glycérine est effectuée par un procédé enzymatique.

**4.** Procédé selon l'une quelconque des revendications 1 à 3, dans lequel une graisse et/ou huile riche en diacylglycérol présentant une teneur en acides gras trans-insaturés parmi les acides gras constitutifs de la graisse et/ou huile de 2 % en poids ou moins est/sont obtenue(s).

**5.** Procédé selon l'une quelconque des revendications 1 à 4, dans lequel la graisse et/ou huile ayant une teneur élevée en diacylglycérol est une graisse et/ou huile contenant des diacylglycérols en une quantité de 50 % en poids ou plus.

**6.** Procédé selon la revendication 1, dans lequel le traitement de désodorisation est effectué de telle sorte que dans le cas de la mise en oeuvre du traitement de désodorisation entre 475 et 480 K, le temps de désodorisation est situé dans la plage allant de 35 à 315 minutes; dans le cas de la mise en oeuvre du traitement de désodorisation entre 480 et 490 K, le temps de désodorisation est situé dans la plage allant de 35 à 210 minutes; dans le cas de la mise en oeuvre du traitement de désodorisation entre 490 et 500 K, le temps de désodorisation est situé dans la plage allant de 30 à 150 minutes; dans le cas de la mise en oeuvre du traitement de désodorisation entre 500 et 510 K, le temps de désodorisation est situé dans la plage allant de 20 à 90 minutes; dans le cas de la mise en oeuvre du traitement de désodorisation entre 510 et 520 K, le temps de désodorisation est situé dans la plage allant de 15 à 52 minutes; dans le cas de la mise en oeuvre du traitement de désodorisation entre 520 et 530 K, le temps de désodorisation est situé dans la plage allant de 12 à 45 minutes; dans le cas de la mise en oeuvre du traitement de désodorisation entre 530 et 540 K, le temps de désodorisation est situé dans la plage allant de 9 à 30 minutes; dans le cas de la mise en oeuvre du traitement de désodorisation entre 540 et 550 K, le temps de désodorisation est situé dans la plage allant de 6 à 22 minutes; et dans le cas de la mise en oeuvre du traitement de désodorisation entre 550 et 563 K, le temps de désodorisation est situé dans la plage allant de 3 à 15 minutes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3007240 B
- JP 2006137923 A
- JP 2007014263 A

**Non-patent literature cited in the description**

- Method for Preparing Fatty Acid Methyl Esters (2.4.1.2-1996). Standard Methods for the Analysis of Fats, Oils and Related Materials